# EUROPEAN PATENT APPLICATION

(11) **EP 4 537 897 A1**
(43) Date of publication of application: **16.04.2025**
(21) Application number: 23823867.9
(22) Date of filing: 12.06.2023
(51) Int. Cl.: A61N 5/067, A61B 18/20

(54) **PHOTOTHERAPY APPARATUS**

(30) Priority: 13.06.2022 JP 2022095070
(71) Applicant: Teijin Pharma Limited, Tokyo 100-0013 (JP)
(72) Inventor: NANJO, Takuya, Tokyo 100-0013 (JP); KAWASE, Yuki, Tokyo 100-0013 (JP); ISHIBASHI, Naoya, Tokyo 100-0013 (JP); OKAYAMA, Takamitsu, Tokyo 100-0013 (JP)
(74) Representative: Hoffmann Eitle
(86) International application number: PCT/JP2023/021654
(87) International publication number: WO 2023/243577

(57) **Abstract**

A phototherapy apparatus comprising a tubular housing a laser light source which is disposed inside the housing and which is configured to e it a laser beam toward a target region from a leading end of the housing; an exhaust port which is provided in a lateral surface of the housing; and a shielding part which is provided on the exterior side of the housing so as to block reflected beams that result from the laser beam unintendedly reflected within the housing and that travel toward the outside through the exhaust port.

## Description

### Technical Field

The present invention relates to a phototherapy apparatus.

### Background Art

There is known a phototherapy apparatus which irradiates a target site which is biological tissue with a laser for the purpose of treatment or treatment assistance, such as for blood circulation promotion and metabolism promotion (Patent Literature 1).

The phototherapy apparatus disclosed in Patent Literature 1 includes a housing having a tubular shape, a laser light source disposed inside the housing and configured to irradiate a target site with a laser from a distal end of the housing, an exhaust port provided in a side surface of the housing, an intake port provided in a side surface of the housing on a side opposite to the exhaust port, and an air supply device configured to supply air into the housing through the intake port.

### Citation List

### Patent Literature

PTL 1: WO 2021/132271

### Summary of Invention

### Technical Problem

In the phototherapy apparatus described in Patent Literature 1, although not anticipated in a normal usage mode, an insertion having a rod shape and including a portion which reflects light, such as a mirror, may be inserted, for example, simply out of curiosity, into the housing through the exhaust port which directly communicates with the outside. In this case, the laser may be reflected outside the housing through the exhaust port. When biological tissue unrelated to the intended treatment or the like is irradiated with reflected light of the laser, there is a risk of adverse effects such as burns on the biological tissue. Further, when an eye is irradiated with the reflected light of the laser, there is a risk of blindness occurring. In particular, primary reflected light (hereinafter referred to as reflected light R) which leaks out after being reflected from a member having high reflectance such as metal or a mirror has a significant impact on a living body, unlike secondary reflected light which leaks out after striking and being reflected from a resin member or the like having low reflectance such as a housing or a shielding member. On the other hand, when the exhaust port is made extremely small or the exhaust port itself is not provided in order to prevent insertion of an insertion, the inside of the housing and even the target site may become very hot.

An object of the present invention is to provide a phototherapy apparatus configured to so that even when a laser is unintentionally reflected inside a housing, the reflected light is not directly visible from the outside.

### Solution to Problem

In an aspect of the present invention, provided is a phototherapy apparatus including a housing having a tubular shape, a laser light source disposed inside the housing and configured to irradiate a target site with a laser from a distal end of the housing, a first opening provided in a side surface of the housing, and a shielding unit provided outside the housing and thus blocking reflected light of the laser unintentionally reflected inside the housing and directed toward an outside through the first opening.

The shielding unit may be provided so that an optical path of the laser emitted from the laser light source is not directly visible from outside the housing through the first opening. The shielding unit may be formed so that when a virtual straight line connecting any point in an optical path range of the laser and any point of an edge portion defining the first opening is drawn, the virtual straight line passes through the shielding unit. The shielding unit may include a shielding wall extending in an axial direction of the housing, and the shielding wall and the housing may form at least one second opening which opens in a circumferential direction of the housing or rearward of the housing. The shielding wall may face the side surface of the housing and curve in a convex shape toward the housing. The phototherapy apparatus may further include a third opening provided in the side surface of the housing and an air supply device configured to supply air into the housing through the third opening.

### Advantageous Effects of Invention

An aspect of the present invention exhibits a common effect of providing a phototherapy apparatus configured so that even if a laser is unintentionally reflected inside a housing, the reflected light is not directly visible from the outside.

### Brief Description of Drawings

[Fig. 1] Fig. 1 is a perspective view of a phototherapy apparatus according to an embodiment of the present invention.
[Fig. 2] Fig. 2 is a perspective view of a shielding unit.
[Fig. 3] Fig. 3 is a longitudinal sectional view of a distal end portion of the phototherapy apparatus.
[Fig. 4] Fig. 4 is a transverse sectional view of the distal end portion of the phototherapy apparatus taken along line A-A in Fig. 3.

### Description of Embodiments

Hereinafter, embodiments of the present invention will be described in detail with reference to the drawings. Corresponding components are denoted by common reference signs throughout the drawings.

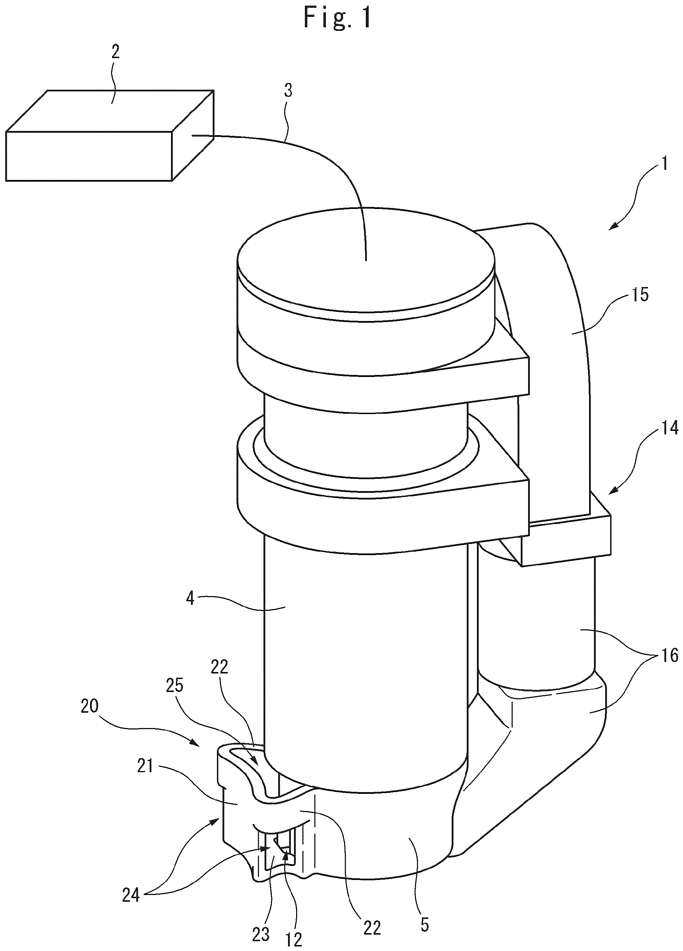

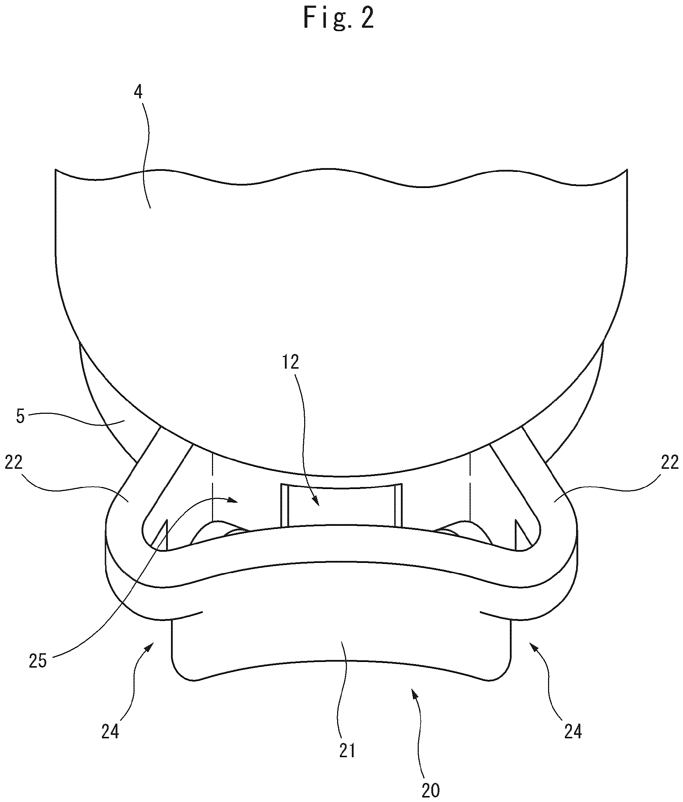

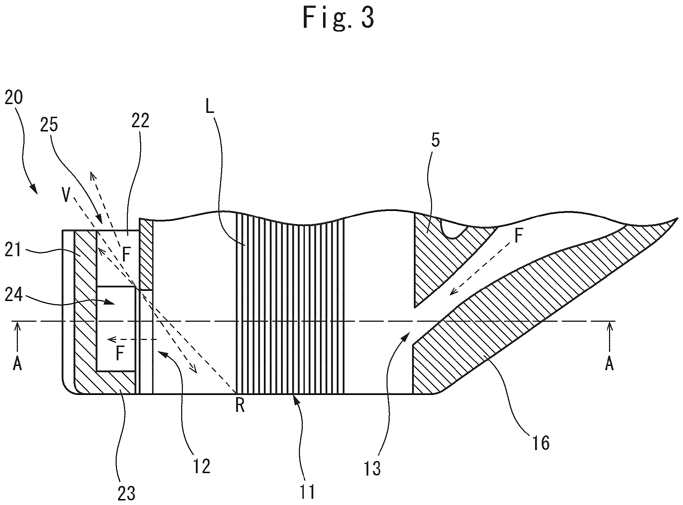

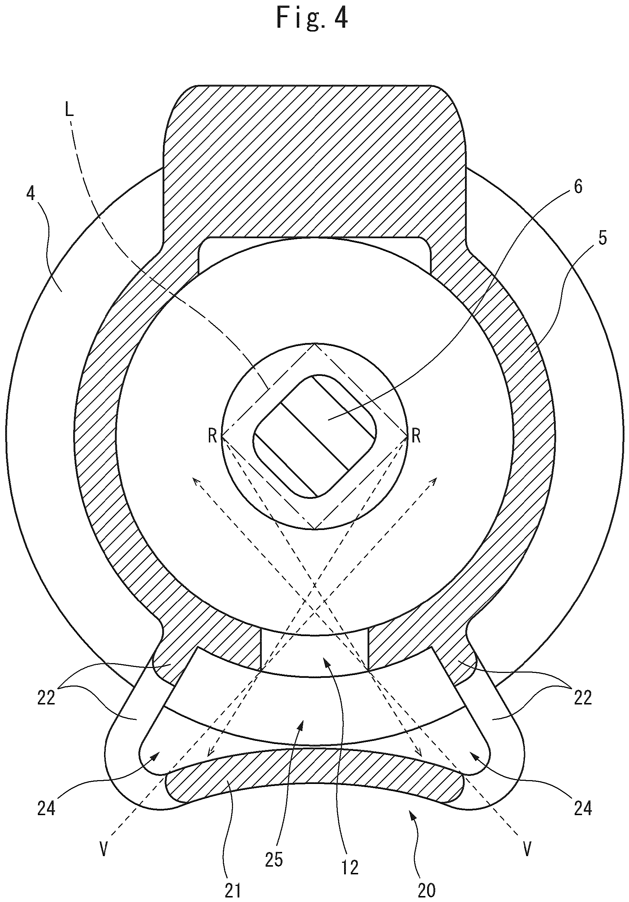

Fig. 1 is a perspective view of a phototherapy apparatus 1 according to an embodiment of the present invention, Fig. 2 is a perspective view of a shielding unit 20, Fig. 3 is a longitudinal sectional view of a distal end portion of the phototherapy apparatus 1, and Fig. 4 is a transverse sectional view of the distal end portion of the phototherapy apparatus 1 taken along line A-A in Fig. 3.

The phototherapy apparatus 1 as a probe is electrically connected to a control device 2 via a cable 3. The control device 2 includes one or more processors, a storage unit, peripheral circuits thereof, and the like. The control device 2 comprehensively controls the overall operation of the phototherapy apparatus 1 on the basis of a computer program stored in advance in a storage unit. At the time of processing, the control device 2 transmits, as appropriate, control signals related to emission and cessation of a laser, start and stopping of a fan, and the like. The control device 2 may include an input/output unit, for example, a display unit such as a display, or an input interface such as an operation button or a touch panel. The phototherapy apparatus 1, the control device 2, the cable 3, the input interface, and the like may be collectively referred to as a phototherapy system.

The phototherapy apparatus 1 includes a housing 4 having a tubular shape and a laser light source 6 disposed inside the housing 4. The housing 4 includes a movable unit 5 having a cylindrical shape disposed at a distal end portion of the housing 4 and movably disposable in an axial direction relative to the housing 4. The housing 4 has a cylindrical shape having a circular cross section as a whole, but may be a member having a tubular shape having an elliptical cross section or may be a member having another shape such as, for example, a tubular shape having a polygonal cross section such as a square or an octagon. A target site is irradiated with the laser emitted from the laser light source 6 through an opening 11 provided in a distal end surface of the housing 4.

The movable unit 5 functions as a contact-type interlock mechanism in the phototherapy apparatus 1. Specifically, the phototherapy apparatus 1 is configured to emit the laser only when the movable unit 5 is retracted by being pressed against the target site or the vicinity of the target site. In other words, the retraction of the movable unit 5 is detected by a sensor disposed inside the housing 4, and the control device 2, triggered by this detection, is configured to enable laser emission. This can prevent unintended laser emission. The phototherapy apparatus 1 need not include the movable unit 5.

An exhaust port 12 which is a first opening is provided on a side surface of the housing 4, specifically, on a side surface of the movable unit 5. An intake port 13 which is a third opening is provided on the side surface of the housing 4, specifically, on the side surface of the movable unit 5, on a side opposite to the exhaust port 12. Positions of the exhaust port 12 and the intake port 13 in the housing 4 may be set as desired in the axial direction and a circumferential direction.

The phototherapy apparatus 1 includes an air supply device 14 which supplies air to be injected into the housing 4 from the intake port 13. The air supply device 14 includes a fan 15 disposed adjacent to the side surface of the housing 4 and a duct 16 connecting the fan 15 and the intake port 13. Accordingly, air F taken in by rotation of the fan 15 is guided to the intake port 13 through the duct 16. The air F supplied into the housing 4 through the intake port 13 removes heat from inside the housing 4 and the target site heated by the laser emission, and is emitted outside the housing 4 through the exhaust port 12. Furthermore, the air F emitted outside the housing 4 is emitted outside the phototherapy apparatus 1 through first vents 24 and a second vent 25 described below. The phototherapy apparatus 1 need not include the air supply device 14 and the intake port 13. In this case, the air F heated by the emission of the laser inside the phototherapy apparatus 1 is emitted outside the housing 4 from the exhaust port 12 by convection. Furthermore, to increase an exhaust efficiency by the convection, a plurality of the exhaust ports 12 may be provided in the housing 4.

As described above, although not anticipated in a normal usage mode, an insertion having a rod shape and including a portion which reflects light, such as a mirror, may be inserted, for example, simply out of curiosity, into the housing 4 through the exhaust port 12. Therefore, the phototherapy apparatus 1 further includes the shielding unit 20 provided integrally with the side surface of the housing 4.

The shielding unit 20 includes a first shielding wall 21 and two second shielding walls 22 extending in the axial direction of the housing 4, and a bottom wall 23 provided flush with the distal end surface of the housing 4. The bottom wall 23 need not be flush with the distal end surface of the housing 4 as long as the bottom wall 23 does not protrude from the distal end surface of the housing 4. The bottom wall 23 may include a bottom surface parallel to the distal end surface of the housing 4 disposed rearward of the distal end surface of the housing 4, or may be an inclined surface inclined rearward from an edge portion of the distal end surface of the housing 4. The first shielding wall 21 is disposed facing the side surface of the housing 4, in particular, the exhaust port 12. A central portion of the first shielding wall 21 in a width direction, i.e., in the circumferential direction, is formed curved in a convex shape toward the housing 4. In other words, the first shielding wall 21 is curved in a direction opposite to the side surface of the housing 4. Each of the two second shielding walls 22 extends radially outward with respect to the axial direction from the side surface of the housing 4. Distal end portions of the two second shielding walls 22 in a radial direction are continuously connected to end portions of the first shielding wall 21 in the circumferential direction. Connection portions between the first shielding wall 21 and the second shielding walls 22 are formed curving outward in a convex shape.

The two connection portions of the first shielding wall 21 and the second shielding walls 22 on the distal end side and the corresponding bottom wall 23 are cut out, defining the two first vents 24. Specifically, the first shielding wall 21 and the second shielding walls 22 are cut out, forming an end surface extending rearward in the axial direction from a distal end side of the housing 4 and an end surface extending in the radial direction at a position away from the distal end of the housing 4. Thus, the two first vents 24, substantially rectangular in shape, disposed on the distal end side of the housing 4, and open in the circumferential direction, are defined in the shielding unit 20. The bottom wall 23 need not be cut out, and only the two connection portions on the distal end side of the first shielding wall 21 and the second shielding wall 22 need be cut out so as to define the first vents 24.

Rear end surfaces of the first shielding wall 21 and the second shielding walls 22 and the side surface of the housing 4 define the second vent 25 disposed on the side surface of the housing 4 and opening rearward. The second vent 25 extends in the circumferential direction and has an elongated shape with a center narrowed by the curvature of the first shielding wall 21. The two first vents 24 and the second vent 25, by the first shielding wall 21, the second shielding walls 22, and the housing 4, constitute a second opening which opens in the circumferential direction of the housing 4 or rearward from the housing 4.

In a use state in which treatment is performed using the phototherapy apparatus 1, a surface of the distal end portion of the phototherapy apparatus 1 including the opening 11 which is the emission port of the laser abuts against the vicinity of the target site. Therefore, the laser is not directly visible through the opening 11 on a distal end side of the phototherapy apparatus 1. On the other hand, in the use state of the phototherapy apparatus 1, the exhaust port 12 is open toward outside the phototherapy apparatus 1. However, a position, a shape, and a size of the shielding unit 20, specifically, each of the first vents 24 and the second vent 25, are set in relation to a position, a shape, and a size of the exhaust port 12 so that an optical path of the laser emitted from the laser light source 6 inside the housing 4 or the reflected light of the laser reflected inside the housing 4 is not directly visible from outside the phototherapy apparatus 1.

In Figs. 3 and 4, an optical path range of the laser is denoted by reference sign L. The optical path range L of the laser refers to a range of an inner space defined by an outermost contour of the emitted laser. In Fig. 4, a range surrounded by a dot dash line is the optical path range L of the laser. As illustrated in Fig. 3, a line of sight V from outside the phototherapy apparatus 1 through the second vent 25 is blocked by the side surface of the housing 4 surrounding the exhaust port 12, and does not reach the optical path range L of the laser. Similarly, as illustrated in Fig. 4, a line of sight V from outside the phototherapy apparatus 1 through the first vents 24 is blocked by the side surface of the housing 4 surrounding the exhaust port 12, and does not reach the optical path range L of the laser.

In short, the shielding unit 20 is provided so that the optical path of the laser emitted from the laser light source 6 is not directly visible from outside the housing 4 through the exhaust port 12. In other words, the shielding unit 20 is at least formed so that when a virtual straight line connecting any point in the optical path range L of the laser and any point of the edge portion defining the exhaust port 12 is drawn, the virtual straight line passes through the shielding unit 20. As a result, even if an insertion having a rod shape and including a portion which reflects light is inserted into the housing 4 from the exhaust port 12 through the first vent 24 or the second vent 25, the laser is not reflected outside the phototherapy apparatus 1.

With reference to Figs. 3 and 4, a case in which an insertion having a curved shape and including a portion which reflects light can be inserted through the exhaust port 12 through the first vent 24 or the second ventilation hole 25, causing reflection of the laser, will be described. In Figs. 3 and 4, the reflected light R directed outward through the exhaust port 12 is blocked by the shielding unit 20, particularly by the first shielding wall 21. Accordingly, the reflected light R is not visible from outside the phototherapy apparatus 1 through the first vents 24 or the second vent 25. In other words, even if an insertion having curved shape and including a portion which reflects light is inserted into the housing 4 from the exhaust port 12 through the first vent 24 or the second vent 25, the laser is not reflected outside the phototherapy apparatus 1.

In short, the shielding unit 20 is configured to deter insertion of an insertion from the exhaust port 12 and, even if such an insertion is inserted, block reflected light of the laser so that the reflected light is not emitted outside. Accordingly, the phototherapy apparatus 1 is configured so that, even if the laser is unintentionally reflected inside the housing 4, the reflected light is not visible from the outside.

The air F emitted radially outward from the exhaust port 12 is guided so as to be emitted outside from the first vents 24 by the first shielding wall 21 curved in a direction opposite to the side surface of the housing 4, i.e., curved in a convex shape toward the housing 4. Furthermore, the air F which remains is emitted outside from the second vent 25. The first shielding wall 21, being curved in the direction opposite to the side surface of the housing 4, can guide the air F more smoothly. The curvature of the first shielding wall 21 may correspond to part of a cylindrical surface with an axial line parallel to an axial line of the housing 4, or may correspond to part of a convex curved surface such as a spherical surface, as long as the curvature can smoothly guide the air F.

In the embodiment described above, the shielding unit 20 is provided integrally with the housing 4, but may be configured separately from the housing 4. In this case, the shielding unit 20 may be configured to be attachable to and detachable from the housing 4. In the embodiment described above, the phototherapy apparatus 1 includes a total of three vents, i.e., the two first vents 24 and the one second vent 25, but may include one or two of these vents, or may additionally include four or more vents. The positions, the shapes, and the sizes of the vents may be determined in accordance with an amount of air supplied from the intake port 13 or the like, in consideration of the reflection of the laser described above and the like. The shielding unit may also be provided not only for the exhaust port 12 but also for another first opening formed for a different purpose.

### Reference Signs List

1 Phototherapy apparatus
2 Control device
3 Cable
4 Housing
5 Movable unit
6 Laser light source
11 Opening
12 Exhaust port
13 Intake port
14 Air supply device
15 Fan
16 Duct
20 Shielding unit
21 First shielding wall
22 Second shielding wall
23 Bottom wall
24 First vent
25 Second vent

## Claims

1. A phototherapy apparatus, comprising:
a housing having a tubular shape;
a laser light source disposed inside the housing and configured to irradiate a target site with a laser from a distal end of the housing;
a first opening provided in a side surface of the housing; and
a shielding unit provided outside the housing and thus blocking reflected light of the laser unintentionally reflected inside the housing and directed toward an outside through the first opening.

2. The phototherapy apparatus according to claim 1, wherein the shielding unit is provided so that an optical path of the laser emitted from the laser light source is not directly visible from outside the housing through the first opening.

3. The phototherapy apparatus according to claim 2, wherein the shielding unit is formed so that when a virtual straight line connecting any point in an optical path range of the laser and any point of an edge portion defining the first opening is drawn, the virtual straight line passes through the shielding unit.

4. The phototherapy apparatus according to claim 3, wherein
the shielding unit includes a shielding wall extending in an axial direction of the housing, and
the shielding wall and the housing form at least one second opening which opens in a circumferential direction of the housing or rearward of the housing.

5. The phototherapy apparatus according to claim 4, wherein the shielding wall faces the side surface of the housing and curves in a convex shape toward the housing.

6. The phototherapy apparatus according to any one of claims 1 to 5, further comprising:
a third opening provided in the side surface of the housing; and
an air supply device configured to supply air into the housing through the third opening.
